# EUROPEAN PATENT SPECIFICATION

(11) **EP 3 473 057 B1**
(45) Date of publication and mention of the grant of the patent: **22.07.2020**
(21) Application number: 17724381.3
(22) Date of filing: 22.05.2017
(51) Int. Cl.: H05B 3/84, A24F 47/00, A61M 11/04

(54) **HEATER ASSEMBLY FOR AN AEROSOL-GENERATING SYSTEM**
HEIZERANORDNUNG FÜR EIN AEROSOLERZEUGUNGSSYSTEM
ENSEMBLE DE CHAUFFAGE POUR SYSTÈME DE GÉNÉRATION D'AÉROSOL

(30) Priority: 20.06.2016 EP 16175298
(43) Date of publication of application: 24.04.2019
(73) Proprietor: Philip Morris Products S.A., 2000 Neuchâtel (CH)
(72) Inventor: DUC, Fabien, 1227 Carouge (CH); TABASSO, Alain, 1417 Essertines-sur-Yverdon (CH)
(74) Representative: Spencer, James Michael
(86) International application number: PCT/EP2017/062303
(87) International publication number: WO 2017/220274

(56) References cited:
- DE-U1- 20 308 376
- US-A1- 2002 078 956
- US-A1- 2007 240 715
- US-A1- 2008 023 003

## Description

The present invention relates to a heater assembly for an aerosol-generating system and an aerosol-generating system comprising the heater assembly.

Handheld electrically operated aerosol-generating systems are known that consist of a device portion comprising a battery and electric circuitry, a cartridge comprising a supply of a liquid aerosol-forming substrate held in a liquid storage portion, and an electrically operated vaporiser. A mesh heater can be utilized as a vaporiser. Such a mesh heater is disclosed, for example, in WO 2015/117702 A1, in which the mesh heater is provided by a plurality of electrically conductive metal filaments. This conventional mesh heater is provided together with the liquid storage portion in the cartridge. The cartridge is often provided as a single-use cartridge, which is disposed - together with the liquid storage portion and the mesh heater - once the aerosol-forming substrate held in the liquid storage portion is depleted. Thus, undesirable waste is created. Also, burning residues can develop on a surface of the mesh heater during repeated use, which could be undesirable burned in a subsequent use of the mesh heater.

US2008/023003A1 describes a hand-operated and portable vaporizing device that, through the application of heat, volatilizes one or more constituents of various vaporizable substances, such as medicines, cigarettes, and plant materials, for the purpose of inhaling these volatilized constituents.

It is desirable to provide a heater assembly which is easy to clean and re-usable, decreasing the cost of the cartridge. Also, it is desirable to provide a heater assembly which reduces the risk of a production of undesirable products and which does not degrade with temperature. Furthermore, it is desirable to achieve a more consistent cartridge-to-cartridge performance.

According to a first aspect of the present invention there is provided a heater assembly for an aerosol-generating system comprising a perforated glass substrate and a heater element, wherein the heater element is provided in or on the glass substrate.

Providing a perforated substrate made of glass has a number of advantages. Glass beneficially has a high heat resistance. Thus, undesirable heating products are prevented when the heater element adjacent to the glass substrate is heated and vaporises liquid aerosol-forming substrate. In other words, the heater element in or on the glass substrate may be heated to relatively high temperatures without damaging the glass substrate and without creating undesirable heating products due to burning of substrate or burning residues on the glass substrate.

As a further advantage, glass is temperature stable such that a multitude of heating and subsequent cooling processes may be performed by the heater element without the glass substrate degrading. Thus, the quality of the aerosol, which is generated by the heater assembly during vaporisation of liquid aerosol-forming substrate, may be maintained at a consistent high quality during repeated use of the heater assembly.

The perforated glass substrate and preferably the heater element may be easily cleaned due to the advantageous surface characteristics of glass. In more detail, glass may have a smooth and hard surface so that the glass substrate is not damaged or harmed during cleaning of the glass substrate. Burning residues and contaminants may be easily cleaned from the surface of the glass substrate.

The perforated glass substrate is provided to enable the liquid aerosol-forming substrate to flow through the glass substrate and to be vaporised by the heater element of the heater assembly. The glass substrate is therefore provided with perforations which have a diameter that allows the liquid aerosol-forming substrate to flow through the glass substrate.

The glass substrate is a generally flat element having any suitable size and shape which allows its incorporation into a handheld system. The area of the glass substrate may be small, preferably less than or equal to 40 square millimeters, 30 square millimeters or 20 square millimeters. The thickness of the glass substrate may be equal or below 2 millimeters, 1 millimeter or 0.5 millimeters.

The glass substrate may have rectangular or circular shape. Preferably the shape of the glass substrate is adapted to the dimensions of the liquid storage portion of the aerosol generating system.

In some embodiments, the perforations of the glass substrate have a width of between 1 micron and 500 microns, preferably between 5 microns to 250 microns, and most preferably between 10 microns and 150 microns.

By providing perforations in the glass substrate with such a width, a suitable amount of liquid aerosol-forming substrate may flow through the glass substrate. The amount of liquid aerosol-forming substrate that may flow through the glass substrate is chosen such that enough aerosol is generated by the heater assembly when a user uses the aerosol-generating system.

The distance between the perforations of the glass substrate is between 1 micron and 1000 microns, preferably between 5 microns to 750 microns, and most preferably between 10 microns and 500 microns.

By providing such a distance between the perforations, a dimensional stability of the glass substrate is provided such that the glass substrate is not prone to breaking during use and cleaning and is not deformed easily. Furthermore, by providing distances between the perforations and perforations of the glass substrate with the above described width, the surface area of the glass substrate is enlarged, while a relatively high dimensional stability of the glass substrate and the heater assembly is provided.

Between 15 to 70 percent, more preferably between 20 to 60 percent and most preferably between 25 to 55 percent of the glass substrate of the heater assembly may be fluid-permeable.

Sufficient amounts of liquid aerosol-forming substrate can thus permeate through the heater assembly to be vaporised, while the heater assembly is dimensionally stable. By providing the heater element of the heater assembly in the form of a mesh or array, the surface area, which can be utilized for vaporising liquid aerosol-forming substrate, may be optimized.

The heater element is provided to vaporise the liquid aerosol-forming substrate. The vaporised liquid aerosol-forming substrate forms an aerosol, which may be inhaled by a user of the aerosol-generating system. Preferably, the heater element is an electric resistance heater, which may be heated by an electric current. Preferably, the heater element is made of an electrically conductive material such as aluminium or copper. Also, any other suitable electrically conductive material could be used as material for the heater element.

The heater element may be provided on a surface of the glass substrate. Preferably, the heater element is provided directly on the surface of the glass substrate. The heater element may be provided as a coating or thin film, preferably a metallic coating or metallic thin film.

The heater element may be provided in the glass substrate. The heater element may be incorporated into the glass substrate such that the heater element is encapsulated or encompassed by the glass substrate. The heater element may be provided as a metallic coating or thin metallic foil sandwiched between two layers of glass substrate. By encapsulating the heater element in the glass substrate or by sandwiching the heater element between two layers of glass substrate, the heater element is substantially protected from contact with the environment and in particular from contact with the liquid aerosol-forming substrate. As the liquid aerosol-forming substrate does not come into direct contact with the hot heater element, but only with the glass substrate, burning residues of liquid aerosol-forming substrate are less likely to be generated on the heater element during or after the vaporisation of the liquid aerosol-forming substrate.

Further, delamination of the metallic thin film is a commonly observed problem in particular for coated ceramic heater elements. By sandwiching the metallic heater thin film in between two layers of glass substrate delaminating of the metallic thin film can efficiently be prevented.

The heater element may extend over the full width of the glass substrate. The heating element may also be provided as an array of linear heating strips or may form a mesh of heating strips. The heating elements could alternatively or additionally also be provided inside the perforations of the glass substrate.

In embodiments, in which the heater elements are provided within the perforations, only, the heater elements may each have contact portions such that electric current may flow through the heater elements and heat the heater elements. If the heater elements are provided in the perforations only, the liquid is directly heated when passing the perforations. In such embodiments it is not necessary to heat the complete glass substrate to elevated temperatures. Instead only the limited amount of liquid that is drawn into the perforations is heated at a time. Accordingly it is expected that significantly less energy is required in evaporation of the liquid aerosol-forming substrate.

The heater assembly may be provided with electrically conductive contact regions for contacting the heating elements provided in or on the glass substrate. The contact regions may be provided in the peripheral area of the glass substrate and may be adapted to establish electric contact to the control unit and the power supply of the aerosol-generating system.

According to a second aspect of the present invention there is provided an aerosol-generating system with a main body and a heater assembly as described above. In some embodiments the main body may comprise the heater assembly. The main body may further comprise a battery, electric circuitry and a mouthpiece. Furthermore, the aerosol-generating system may be adapted to accommodate a replaceable or refillable liquid storage portion. The liquid storage portion may be provided attachable to the main body. The main body may comprise a sensor, preferably a flow sensor, to detect when a user draws on the aerosol-generating system. Following this detection, the electric circuitry controls a flow of electric current from the battery through the heater element.

The heater assembly may be provided as an integral part of the main body. The aerosol-generating system is configures such that in the assembled state the heater assembly is located adjacent to and in fluid communication with the liquid storage portion.

The liquid storage portion may comprise a housing having an opening, which may be sealed prior use by a suitable sealing element. The sealing element may be a foil that can be removed by the user before or upon insertion of the liquid storage portion into the aerosol-generating system. The liquid storage portion further comprises liquid aerosol-forming substrate that may fully or partially be absorbed in a capillary element, provided within the liquid storage portion. The capillary element is preferably disposed within the liquid storage portion in direct vicinity to the opening of the housing of the liquid storage portion.

When inserted into the aerosol-generating system, the opening of the liquid storage portion is located adjacent to the heater assembly, such that the capillary element of the liquid storage portion is in direct contact with the heater assembly. In this way it is ensured that the liquid aerosol-forming substrate may be conveyed from the liquid storage portion through the capillary element towards the heater assembly. Thus, the heater element is moisturized with liquid aerosol-forming substrate.

Suitable capillary materials may have a spongy or fibrous structure, which allows the liquid aerosol-forming substrate to be conveyed from the liquid storage portion to the heater assembly by capillary action. The capillary material may prevent that liquid aerosol-forming substrate leaks out of the liquid storage portion.

The liquid storage portion may be part of a cartridge, wherein the cartridge comprises the liquid storage portion and a mouthpiece. The cartridge may be provided as a single-use cartridge which is disposed when the liquid aerosol-forming substrate in the liquid storage portion is depleted. Alternatively, the liquid storage portion may be a refillable liquid storage portion.

The mouthpiece portion may be connected to the main body of the device by a hinged connection. This ensures that the mouthpiece portion and main body stay together, reducing the chance of a user losing one of the mouthpiece portion or the main body. It also provides for simple insertion of the liquid storage portion into the device and removal of the cartridge from the device. The mouthpiece portion may be retained in a closed position by a clasp mechanism. The clasp mechanism may comprise a release button and may be configured to release the mouthpiece portion when the release button is depressed. The mouthpiece portion may retained in a closed position by other mechanisms such as a magnetic closure or a by using a bi-stable hinge mechanism. However, other means of connection of the mouthpiece portion to the main body are possible, such as screw-fitting or snap-fitting.

The mouthpiece portion may include an air inlet and an air outlet and may be configured to allow a user to suck on the air outlet to draw air through the mouthpiece portion from the inlet to the outlet. The mouthpiece portion may include a baffle configured to direct air drawn through the mouthpiece portion from the inlet to the outlet past the vaporiser in the cartridge. By keeping all the airflow within the mouthpiece portion, the design of the main body can be made very simple. It also means that only the mouthpiece portion of the device need be cleaned or replaced after prolonged use. However, it should be clear that other airflow patterns are possible and that other orientations of the liquid storage portion within the device are possible.

The heater element may be disposed in the mouthpiece of the main body. By providing the heater element in the mouthpiece of the main body, the heater element is provided directly adjacent to the liquid storage portion, when the liquid storage portion is connected to the main body. The liquid aerosol-forming substrate from the liquid storage portion may then be easily conveyed from the liquid storage portion to the heater element and subsequently be inhaled by a user through the mouthpiece.

According to a third aspect of the present invention there is provided a process for manufacturing a heater assembly for an aerosol-generating system. The process comprises the step of providing a perforated glass substrate and providing a heater element in or on the glass substrate.

The perforated glass substrate may be manufactured and thus provided by a phase separation process, a sintering process or a sol-gel process.

When the heater element is provided on the glass substrate, the heater element may be provided as an electrically conductive thin film. Thus the glass substrate is coated with the heater element such that the heater element is provided directly on the surface of the glass substrate. Consequently, the heater element may be provided with essentially the same dimensions as the glass substrate. In other words, the heater element may comprise perforations with essentially the same dimensions as described above with reference to the glass substrate. Furthermore, the heater element may be fluid permeable as described above with reference to the glass substrate.

When the heater element is provided in the glass substrate, preferably a first portion of the glass substrate is provided. Then, the heater element is provided on the first portion of the glass substrate, preferably as a thin film. Subsequently, a second portion of the glass substrate is provided on the first portion and on the heater element. Thus, the first and second portions of the glass substrate together encapsulate or encompass the heater element. Preferably, only contact portions of the heater element are not encapsulated by the glass substrate. In other words, the heater element may be sandwiched between the first and second portion of the glass substrate. In the perforations of the glass substrate, the heater element may be exposed such that liquid aerosol-forming substrate may be vaporised in the perforations. In more detail, the heater element may be sandwiched between the first and second portion of the glass substrate such that the heater element only comes into direct contact with the liquid aerosol-forming substrate within the perforations. Thus, the heater element may be directly exposed to the liquid aerosol-forming substrate within the perforations, while the rest of the heater element is isolated from the liquid aerosol-forming substrate by the first and second portion of the glass substrate.

Features described in relation to one aspect may equally be applied to other aspects of the invention.

Embodiments of the invention will now be described, by way of example only, with reference to the accompanying drawings, in which:
Figure 1 shows three embodiments of the heater assembly of the present invention;
Figure 2 shows a perforation of an embodiment of the heater assembly of the present invention;
Figure 3 shows a sectional view of an embodiment of the heater assembly of the present invention with a liquid storage portion; and
Figure 4 is a sectional view of an embodiment of an aerosol-generating system according to the present invention.

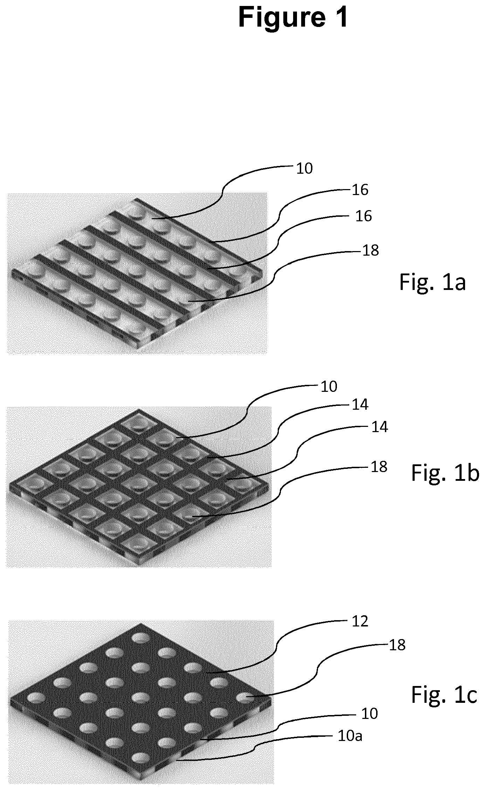

Fig.1 shows three embodiments of the heater assembly of the present invention. The heater assembly comprises a perforated glass substrate 10 and a heater element 12, 14, 16.

The glass substrate 10 is generally a flat rectangular element having a size of 5 x 5 square millimetres and a thickness of 1 millimeter. The small through holes or perforations 18 are provided in a regular pattern across the surface area of the glass substrate 10. The perforations 18 have a width of 50 microns and are separated from each other by 50 microns.

The heating element 12, 14, 16 is provided between the perforations 18. In Fig. 1a the heating element 12, 14, 16 is provided as parallel strips 16 of a conductive metallic thin film that are provided between alternating rows of perforations 18.

In Fig. 1b the heating element 12, 14, 16 is provided in the form of parallel strips and transversal strips 14 of a conductive metallic thin film that form a heating mesh or heating grid wherein each cell of the mesh comprises one perforation 18.

In Fig. 1c the heating element 12, 14, 16 is provided in the form of a uniform thin film 12 provided on the surface area of the glass substrate 10 surrounding the perforations 18.

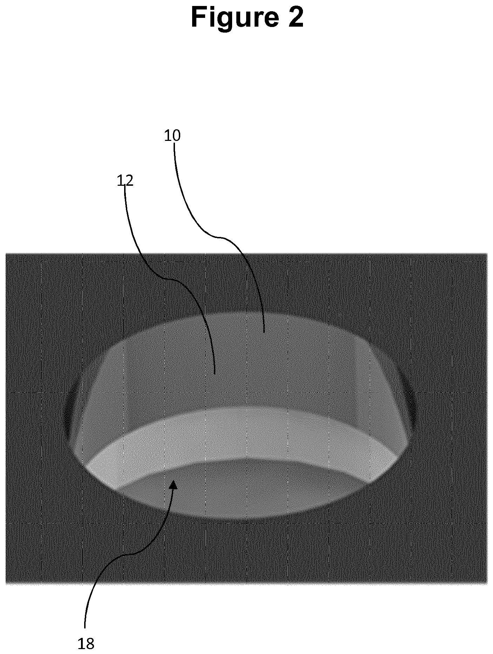

Fig. 2 is an enlarged view of a single perforation 18. As depicted in Fig. 2 the metallic heating thin film may also extend within the periphery of the perforations 18.

In Figs. 1 and 2 the heater element 12, 14, 16 is provided on a surface of the glass substrate 10. Alternatively the heater element 12, 14, 16 may also be provided within the glass substrate 10. One way to provide the heater element 12, 14, 16 within the glass substrate 10 is to sandwich the heater element 12, 14, 16 between two layers of glass substrate 10, 10a. In embodiments in which the heater element 12, 14, 16 does not extend completely to the peripheries of the perforations 18, the heater assembly may be configured such that contact between the heater element 12, 14, 16 and the liquid aerosol-forming substrate may be avoided or at least substantially reduced.

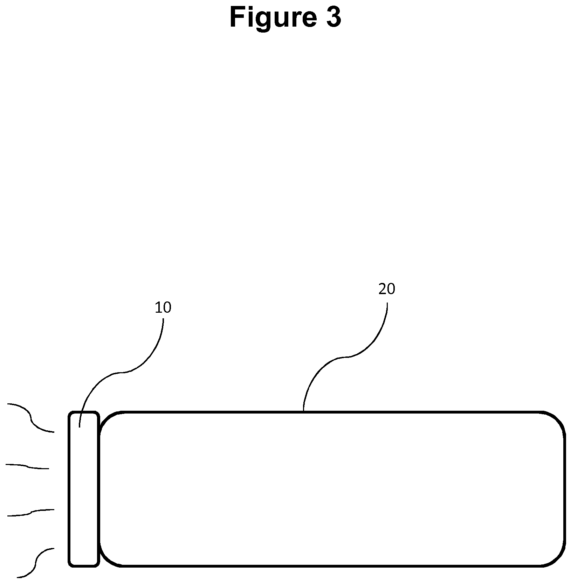

In use the perforated heater assembly is brought into contact with a liquid storage portion 20 as depicted in Fig. 3. The liquid storage portion 20 may comprise a capillary material that contacts the heater assembly ensuring that the liquid aerosol-forming substrate is conveyed from the liquid storage portion 20 through the capillary element towards the heater assembly. Thus, the heater element 12, 14, 16 is at any time moisturized with liquid aerosol-forming substrate. Upon application of electric power to the heater element 12, 14, 16, the heater assembly is heated and the liquid aerosol-forming substrate is evaporated eventually forming an aerosol that may be inhaled by the user.

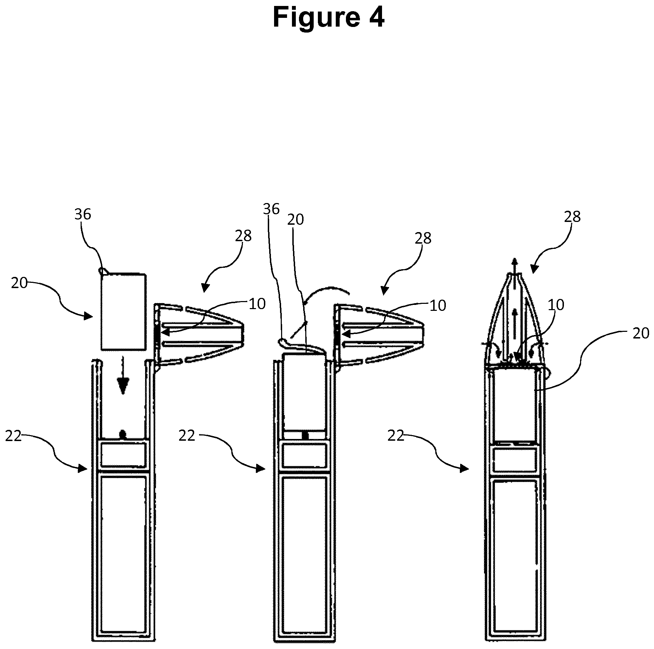

Fig. 4 shows an aerosol-generating system which comprises a main body 22. The main body 22 comprises electric circuitry 24 and a battery 26. Furthermore, the main body 22 comprises the heater assembly as described above. The battery 26 is electrically connected to the heater element 12, 14, 16 by means of contact portions of the heater element 12, 14, 16. The electric circuitry 24 controls the flow of electric current from the battery 26 to the heater element 12, 14, 16 such that the heater element 12, 14, 16 is heated when a user activates the aerosol-generating system. In order to activate the aerosol-generating system, the aerosol-generating system can comprise a flow sensor, which detects when a user draws on the aerosol-generating system.

In Fig. 4, the heater element 12, 14, 16 is disposed in a mouthpiece 28. The mouthpiece 28 is provided as a part of the main body 22. The mouthpiece 28 is connected to the main body 22 by a hinged connection and can move between an open position and a closed position as shown in Fig. 4. The mouthpiece portion 28 is placed in the open position to allow for insertion and removal of a liquid storage portion 20 and is placed in the closed position when the system is to be used to generate aerosol. The mouthpiece 28 comprises a plurality of air inlets 30 and an outlet 32. In use, a user sucks or puffs on the outlet 32 to draw air from the air inlets 30, through the mouthpiece 28 to the outlet 32, and thereafter into the mouth or lungs of the user. Internal baffles 34 are provided to force the air flowing through the mouthpiece 28 past the liquid storage portion 20. The internal baffles 34, which are integrally moulded with the external walls of the mouthpiece 28 ensure that, as air is drawn from the inlets 30 to the outlet 32, it flows over the heater assembly where liquid aerosol-forming substrate is being vaporised. As the air passes the heater assembly, vaporised substrate is entrained in the airflow and cools to form an aerosol before exiting the outlet 32. Accordingly, in use, the aerosol-forming substrate passes through the heater assembly by passing through the perforations 18 in the glass substrate 10 and the heater element 12, 14, 16 as it is vaporised.

The liquid storage portion 20 contains the liquid aerosol-forming substrate. The liquid storage portion 20 is replaceable. The liquid storage portion 20 is replaced once the liquid aerosol-forming substrate in the liquid storage portion 20 is depleted.

The aerosol-generating system can be activated by a sensor in the main body 22 which senses a negative pressure due to the user drawing on the mouthpiece 28. Then, the electric circuitry 24 controls a flow of electric current from the battery 26 to the heater element 12, 14, 16 of the heater assembly.

The liquid storage portion 20 comprises a sealing foil 36 which is removed before inserting the liquid storage portion 20 into the main body 22. The sealing foil 36 prevents that the liquid aerosol-forming substrate leaks out of the liquid storage portion 20 before the liquid storage portion 20 is fully inserted into the main body 22 and the mouthpiece 28 is pivoted back in the closed position.

The exemplary embodiments described above illustrate but are not limiting. In view of the above discussed exemplary embodiments, other embodiments consistent with the above exemplary embodiments will now be apparent to one of ordinary skill in the art.

### Reference signs

- 10, 10a: glass substrate
- 12, 14, 16: heater element
- 18: perforations
- 20: liquid storage portion
- 22: main body
- 24: electric circuitry
- 26: battery
- 28: mouthpiece
- 30: air inlets
- 32: air outlet
- 34: internal baffles
- 36: sealing foil

## Claims

1. Heater assembly for an aerosol-generating system comprising a glass substrate (10) and a heater element (12, 14, 16), wherein the heater element (12, 14, 16) is provided in or on the glass substrate (10), and **characterised in that** the glass substrate (10) is a perforated glass substrate (10).

2. The heater assembly according to claim 1, wherein the perforations (18) have a width of between 1 microns and 500 microns, preferably between 5 microns to 250 microns, and most preferably between 10 microns and 150 microns.

3. The heater assembly according to claim 2, wherein the distance between the perforations (18) of the glass substrate (10) is between 1 micron and 1000 microns, preferably between 5 microns to 750 microns, and most preferably between 10 microns and 500 microns.

4. The heater assembly according to any one of the preceding claims, wherein the heater element (12, 14, 16) or the perforations (18) of the glass substrate (10) or the heater element (12, 14, 16) and the perforations (18) of the glass substrate (10) is/are arranged as a mesh or array.

5. The heater assembly according to claim 4, wherein the area of the mesh or array is less than or equal to 25 square millimetres, preferably 15 square millimetres.

6. The heater assembly according to claim 4 or 5, wherein between 25 and 56 percent of the surface area of the mesh or array is fluid-permeable.

7. The heater assembly according to any one of the preceding claims, wherein the length of the heater assembly is 3 millimetres and the width of the heater assembly is 5 millimetres.

8. The heater assembly according to any one of the preceding claims, wherein the heater element (12, 14, 16) is provided as a thin film on the glass substrate (10).

9. The heater assembly according to any one of the preceding claims, wherein the heater element (12, 14, 16) is provided incorporated into the glass substrate (10) such that the heater element (12, 14, 16) is isolated from an adjacent aerosol-forming substrate.

10. The heater assembly according to any one of the preceding claims, wherein the heater element (12, 14, 16) is provided within the perforations (18).

11. The heater assembly according to any one of the preceding claims, wherein the heater assembly element comprises two perforated glass substrates (10, 10a) and wherein the heater element (12, 14, 16) is provided in between the two perforated glass substrates (10, 10a).

12. Aerosol-generating system comprising:
a main body (22) with a battery (26), electric circuitry (24) and preferably a mouthpiece (28); and
a replaceable or refillable liquid storage portion (20), wherein the liquid storage portion (20) is provided attachable to the main body (22),
wherein the main body (22) further comprises a heater assembly according to any one of the preceding claims.

13. The aerosol-generating system according to claim 12, wherein the heater element (12, 14, 16) is disposed adjacent to the liquid storage portion (20) when the liquid storage portion (20) is attached to the main body (22), such that the aerosol-forming substrate in the liquid storage portion (20) can be vaporised by heating the heater element (12, 14, 16).

14. The aerosol-generating system according to any one of claims 12 or 13, wherein the heater element (12, 14, 16) is disposed in a mouthpiece (28) of the main body (22).

15. Process for manufacturing a heater assembly for an aerosol-generating system, comprising the following steps:
providing a perforated glass substrate (10); and
providing a heater element (12, 14, 16) in or on the glass substrate (10).

## Patentansprüche

1. Heizvorrichtungsbaugruppe für ein Aerosolerzeugungssystem, aufweisend ein Glassubstrat (10) und ein Heizelement (12, 14, 16), wobei das Heizelement (12, 14, 16) in oder auf dem Glassubstrat (10) vorgesehen ist, und **dadurch gekennzeichnet, dass** das Glassubstrat (10) ein perforiertes Glassubstrat (10) ist.

2. Heizvorrichtungsbaugruppe nach Anspruch 1, wobei die Perforationen (18) eine Breite zwischen 1 Mikrometer und 500 Mikrometer, bevorzugt zwischen 5 Mikrometer und 250 Mikrometer und am meisten bevorzugt zwischen 10 Mikrometer und 150 Mikrometer aufweisen.

3. Heizvorrichtungsbaugruppe nach Anspruch 2, wobei der Abstand zwischen den Perforationen (18) des Glassubstrats (10) zwischen 1 Mikrometer und 1000 Mikrometer, bevorzugt zwischen 5 Mikrometer und 750 Mikrometer und am meisten bevorzugt zwischen 10 Mikrometer und 500 Mikrometer beträgt.

4. Heizvorrichtungsbaugruppe nach einem der vorstehenden Ansprüche, wobei das Heizelement (12, 14, 16) oder die Perforationen (18) des Glassubstrats (10) oder das Heizelement (12, 14, 16) und die Perforationen (18) des Glassubstrats (10) als ein Netz oder Array angeordnet ist/sind.

5. Heizvorrichtungsbaugruppe nach Anspruch 4, wobei die Fläche des Netzes oder Arrays kleiner oder gleich 25 Quadratmillimeter, bevorzugt 15 Quadratmillimeter, ist.

6. Heizvorrichtungsbaugruppe nach Anspruch 4 oder 5, wobei zwischen 25 und 56 Prozent des Flächenbereichs des Netzes oder Arrays fluiddurchlässig ist.

7. Heizvorrichtungsbaugruppe nach einem der vorstehenden Ansprüche, wobei die Länge der Heizvorrichtungsbaugruppe 3 Millimeter und die Breite der Heizvorrichtungsbaugruppe 5 Millimeter beträgt.

8. Heizvorrichtungsbaugruppe nach einem der vorstehenden Ansprüche, wobei das Heizelement (12, 14, 16) als Dünnfilm auf dem Glassubstrat (10) vorgesehen ist.

9. Heizvorrichtungsbaugruppe nach einem der vorstehenden Ansprüche, wobei das Heizelement (12, 14, 16) in das Glassubstrat (10) integriert vorgesehen ist, sodass das Heizelement (12, 14, 16) von einem benachbarten aerosolbildenden Substrat isoliert ist.

10. Heizvorrichtungsbaugruppe nach einem der vorstehenden Ansprüche, wobei das Heizelement (12, 14, 16) innerhalb der Perforationen (18) vorgesehen ist.

11. Heizvorrichtungsbaugruppe nach einem der vorstehenden Ansprüche, wobei das Heizvorrichtungsbaugruppenelement zwei perforierte Glassubstrate (10, 10a) aufweist, und wobei das Heizelement (12, 14, 16) zwischen den beiden perforierten Glassubstraten (10, 10a) vorgesehen ist.

12. Aerosolerzeugungssystem, aufweisend:
einen Hauptkörper (22) mit einer Batterie (26), elektrischen Schaltungen (24) und bevorzugt einem Mundstück (28); und
einen auswechselbaren oder nachfüllbaren Flüssigspeicherteil (20), wobei der Flüssigspeicherteil (20) an dem Hauptkörper (22) anbringbar vorgesehen ist,
wobei der Hauptkörper (22) weiter eine Heizvorrichtungsbaugruppe nach einem der vorstehenden Ansprüche aufweist.

13. Aerosolerzeugungssystem nach Anspruch 12, wobei das Heizelement (12, 14, 16) neben dem Flüssigspeicherteil (20) angeordnet ist, wenn der Flüssigspeicherteil (20) an dem Hauptkörper (22) angebracht ist, sodass das aerosolbildende Substrat in dem Flüssigspeicherteil (20) durch Erwärmen des Heizelements (12, 14, 16) verdampft werden kann.

14. Aerosolerzeugungssystem nach einem der Ansprüche 12 oder 13, wobei das Heizelement (12, 14, 16) in einem Mundstück (28) des Hauptkörpers (22) angeordnet ist.

15. Verfahren zum Herstellen einer Heizvorrichtungsbaugruppe für ein Aerosolerzeugungssystem, aufweisend die folgenden Schritte:
Vorsehen eines perforierten Glassubstrats (10); und
Vorsehen eines Heizelements (12, 14, 16) in oder auf dem Glassubstrat (10).

## Revendications

1. Ensemble de chauffage pour un système de génération d'aérosol comprenant un substrat de verre (10) et un élément de chauffage (12, 14, 16), dans lequel l'élément de chauffage (12, 14, 16) est fourni dans ou sur le substrat de verre (10), et **caractérisé en ce que** le substrat de verre (10) est un substrat de verre perforé (10).

2. Ensemble de chauffage selon la revendication 1, dans lequel les perforations (18) ont une largeur comprise entre 1 microns et 500 microns, de préférence entre 5 microns et 250 microns, et le plus préférablement entre 10 microns et 150 microns.

3. Ensemble de chauffage selon la revendication 2, dans lequel la distance entre les perforations (18) du substrat de verre (10) est comprise entre 1 micron et 1000 microns, de préférence entre 5 microns et 750 microns, et le plus préférablement entre 10 microns et 500 microns.

4. Ensemble de chauffage selon l'une quelconque des revendications précédentes, dans lequel l'élément de chauffage (12, 14, 16) ou les perforations (18) du substrat de verre (10) ou l'élément de chauffage (12, 14, 16) et les perforations (18) du substrat de verre (10) sont disposés en forme de maille ou en série.

5. Ensemble de chauffage selon la revendication 4, dans lequel la zone de la maille ou de la série est inférieure ou égale à 25 millimètres carrés, de préférence 15 millimètres carrés.

6. Ensemble de chauffage selon la revendication 4 ou 5, dans lequel entre 25 et 56 pour cent de la surface de la maille ou de la série est perméable aux fluides.

7. Ensemble de chauffage selon l'une quelconque des revendications précédentes, dans lequel la longueur de l'ensemble de chauffage est de 3 millimètres et la largeur de l'ensemble de chauffage est de 5 millimètres.

8. Ensemble de chauffage selon l'une quelconque des revendications précédentes, dans lequel l'élément de chauffage (12, 14, 16) est fourni sous la forme d'un film mince sur le substrat de verre (10).

9. Ensemble de chauffage selon l'une quelconque des revendications précédentes, dans lequel l'élément de chauffage (12, 14, 16) est fourni incorporé dans le substrat de verre (10) de telle sorte que l'élément de chauffage (12, 14, 16) est isolé d'un substrat formant un aérosol adjacent.

10. Ensemble de chauffage selon l'une quelconque des revendications précédentes, dans lequel l'élément de chauffage (12, 14, 16) est fourni dans les perforations (18).

11. Ensemble de chauffage selon l'une quelconque des revendications précédentes, dans lequel l'élément de l'ensemble de chauffage comprend deux substrats de verre perforés (10, 10a) et dans lequel l'élément de chauffage (12, 14, 16) est fourni entre les deux substrats de verre perforés (10, 10a).

12. Système de génération d'aérosol comprenant :
un corps principal (22) avec une batterie (26), un circuit électrique (24) et de préférence une embout buccal (28) ; et
une partie de stockage de liquide remplaçable ou rechargeable (20), dans lequel la partie de stockage de liquide (20) est fournie fixée au corps principal (22),
dans lequel le corps principal (22) comprend en outre un ensemble de chauffage selon l'une quelconque des revendications précédentes.

13. Système de génération d'aérosol selon la revendication 12, dans lequel l'élément de chauffage (12, 14, 16) est disposé à côté de la partie de stockage de liquide (20) lorsque la partie de stockage de liquide (20) est fixée au corps principal (22), de telle sorte que le substrat formant aérosol dans la partie de stockage de liquide (20) peut être vaporisé en chauffant l'élément de chauffage (12, 14, 16).

14. Système de génération d'aérosol selon l'une quelconque des revendications 12 ou 13, dans lequel l'élément de chauffage (12, 14, 16) est disposé dans un embout buccal (28) du corps principal (22).

15. Procédé de fabrication d'un ensemble de chauffage pour un système de génération d'aérosol, comprenant les étapes suivantes :
la fourniture d'un substrat de verre perforé (10) ; et
la fourniture d'un élément de chauffage (12, 14, 16) dans ou sur le substrat de verre (10).
